# EUROPEAN PATENT APPLICATION

(11) **EP 3 473 275 A1**
(43) Date of publication of application: **24.04.2019**
(21) Application number: 18201195.7
(22) Date of filing: 18.10.2018
(51) Int. Cl.: A61K 49/18

(54) **BIOSTABLE MARKER ACTIVE IN NUCLEAR MAGNETIC RESONANCE AND THE USE OF A BIOSTABLE MARKER IN MAGNETIC RESONANCE IMAGING**

(30) Priority: 18.10.2017 PL 42318717
(71) Applicant: Instytut Fizyki Pan, 02-668 Warszawa (PL)
(72) Inventor: Kaszewski, Jaroslaw, 02-760 Warszawa (PL); Godlewski, Marek, 03-543 Warszawa (PL)
(74) Representative: Witek, Rafal

(57) **Abstract**

The first subject of the invention is a biostable nuclear magnetic resonance marker comprising a carrier substance and an active substance, characterized in that the carrier substance is zirconium oxide (ZrO₂) nanoparticles obtained by the microwave solvothermal method and/or zinc oxide nanoparticles (ZnO), whereas the active substance is a solvent, wherein it contains the active substance in an amount of from 0.5% by mass of the marker to 10% by mass of the marker. A second object of the invention is the use of a biostable marker as a contrast agent in magnetic resonance imaging.

## Description

The subject of the invention is a biostable marker active in nuclear magnetic resonance and the use of a biostable marker in magnetic resonance imaging. This marker is intended for use in diagnosing living organisms using devices that use the phenomenon of nuclear magnetic resonance imaging (MRI).

Currently, there are many contrast agents available for MRI diagnostics on the market. Nevertheless, the vast majority of them are various chelates of gadolinium ions, for example as described in the publication Zhuxian Zhou and Zheng-Rong Lu. Gadolinium-Based Contrast Agents for MR Cancer Imaging. Wiley Interdiscip Rev Nanomed Nanobiotechnol. 2013 January; 5 (1): 1-18. doi: 10.1002 / wnan.1198*.* Despite their undoubted advantages as contrast agents gadolinium derivatives have several serious disadvantages and limitations. The first and the most serious problem associated with them is the strong toxicity of free gadolinium ions. Free Gd ions accumulate in the liver, spleen, bones, and the LD50 determined for mice was ∼ 0.2 mmol/kg. *[*M.F. Tweedle, V.M. Runge, Gadoteridol. Drugs Future 17 (1992)187*.], [*W.P. Cacheris, S.C. Quaryz S.M. Rocklage, Magn. Resort. Imaging 8 (1990) 467.] There are also known markers in which the active substance (contrast agent) are manganese ions. The main disadvantages of using manganese as a contrast agent are difficulties in generating stable complexes in which Mn²⁺ ion will not be displaced by endogenous substrates. An additional complication is a passing of a manganese through the calcium channels of organisms. Indeed, this feature allows to use it in imaging dynamic changes in the nervous system, but carries the risk of harmful effects on the brain. Markers based on various types of iron oxides are also known. They are the longest used markers and belong to the most popular contrasting compounds with the use of T₂ transverse relaxation time. Their decisive advantage is the low toxicity associated with the presence of iron in the body under physiological conditions. This advantage is also a disadvantage, because the imaging has limited contrast efficiency and their dark signal is often confused with the low level of the signal of adjacent tissues, such as bones or vessels. In the publication: Lam T, Pouliot P, Avti PK, et al. Superparamagnetic iron oxide based nanoprobes for imaging and theranostics. Adv Colloid Interface Sci. 2013;199-200:95-113*.* are described two iron oxides most often used in biomedical applications, they are: magnetite (Fe₃O₄) and its oxidized and more stable form - maghemite (g-Fe₂O₃). The critical maximum size of superparamagnetic observations is about 25 nm for magnetite and 30 nm for maghemite. Because these two iron oxides exhibit superparamagnetic behavior, in the absence of an external magnetic field they do not tend to self-aggregate, which helps to achieve a good biological response. Very interesting alternative that has recently emerged in the field of contrast agents affecting the T₂ relaxation time is the use of Dy³⁺ ions. Dysprosium is used in the form of chelates or nanoparticles. It has excellent contrast properties in resonances with high magnetic fields (3-9.4 T), which is related to the high magnetic moment of this element. Unlike commonly used nanomaterials dysprosium compounds have many times higher magnetic contrast properties with an induction of more than 3 tesla, whereas most of the used contrast agents have their optimum at around 1.5 to 3 tesla, but have not been tested in vivo. [P.V. Baptista, P. Quaresma, R. Franco, Nanoparticles in molecular diagnostics, Prog. Moth. Biol. Transl. Sci.104 (2011) 427-488*.]* Intensive researches on such a modification of contrast agents, i.e. markers, especially based on nanotechnology are under way. In these studies, particular emphasis is placed on the development of such nanoparticles that would show both a positive T₁ contrast and a negative T₂ contrast. The promising model are ultra-small magnetic iron oxide nanoparticles with a diameter of approximately 3.3 ± 0.5 nm. These nanoparticles, what have been studied, are characterized by very good contrasting properties of anatomical details in the mouse model, as described in the publication: Zhen U, Pei Wei Yi, Qiao Sun, Hao Lei, Hong U Zhao, Zhong Hua Zhu Sean C. Smith, Min Bo Lan' Gao Qing (Max) Lu. Ultrasmall Water-Soluble and Biocompatible Magnetic Iron Oxide Nanoparticles as Positive and Negative Dual Contrast Agents Adv. Funct. Mater.2012, DOI: 10.1002/adfm.201103123*.* In the described experiment, however, a specialized MRI apparatus was used with a magnet of induction of 4.7 tesla, whereas the apparatus used as standard in diagnostics have magnets with magnetic induction between 1.5 and 3T. Therefore, it is not known whether the results will be reproducible on typical MRI devices.

The technical problem to deal with the invention is to provide such a marker that can be in the form of a suspension or tablet, will be show high contrast in imaging with nuclear magentic resonance at a low content of active ingredient, can easily be obtained and maintain biological inertness. Unexpectedly, the above problems were solved by the present invention.

The first subject of the invention is a biostable marker active in nuclear magnetic resonance in the form of a powder, tablet or suspension, containing a carrier substance and an active substance characterized in that the carrier substance is obtained by the microwave solvothermal method zirconium oxide nanoparticles (ZrO₂) and/or zinc oxide nanoparticles (ZnO), while the active substance is a solvent, wherein it contains the active substance in an amount from 0.5% m/m of the marker to 10% m/m of the marker.

Preferably, the carrier substance is obtained by the microwave solvothermal method zirconium oxide nanoparticles (ZrO₂) and/or zinc oxide nanoparticles (ZnO) and lanthanide ions, preferably such as: La³⁺, Ce³⁺, Pr³⁺, Nd³⁺, Sm³⁺, Eu³⁺, Tb³⁺, Ho³⁺, Er³⁺, Tm³⁺, Yb³⁺, Lu³⁺ in an amount of 0.01% to 20 mole%.

Preferably, the lanthanide ion is selected from the group consisting of: Eu³⁺, Pr³⁺, Tb³⁺, Ce³⁺.

Preferably, the marker is obtained by a microwave-assisted solvothermal method from a transition metal solution having a concentration of a transition metal in a solvent solution in the range from 0.001 mol/m³ to 5 mol/dm³, the transition metal solution also contains ammonia water and solvent, and a solution pH of 6 to 11, and the sludge obtained from the solution is rinsed with a solvent, and the microwave-assisted solvothermal process is carried out in the pressure range from 2MPa to 10MPa in a time not shorter than 1s.

Preferably the particle size of the powder is from 1 nm to 100 nm.

Preferably, the solvent is selected from the group consisting of: water, alcohol or physiological saline solution.

Preferably, the minimum powder concentration in the suspension is 0.001 µg/L.

A second aim of the invention is the use of a biostable marker as a contrast in magnetic resonance imaging.

The marker according to the invention is bio-neutral because it contains oxide nanoparticles, biologically inert, chemically resistant, forming stable in real time suspensions in water, alcohols, benzene, isotonic solutions. In addition, this marker is cheap to produce and simple to manufacture, it is characterized by a contrast at the level of gadolinium-based markers. The components of the label are bio-neutral, in contrast to toxic gadolinium, which can be released from the marker and accumulated in the nervous system. In addition, from the biological point of view it has the following advantages: non-toxicity, ease of administration, biodegradability (no need to remove after diagnosis, as in the case of ZnO), biostability (beneficial in the case of multi-stage diagnostics, only ZrO₂). In addition, the marker according to the invention can be modified so that, in addition to the MRI before surgery, it can be used as an auxiliary fluorescent contrast intraoperatively - this is not possible with the use of MRI or CT. The markers of the invention can be modified in such a way that one administration to the patient allows MRI imaging and a therapy with nanoparticles (e.g., photodynamic therapy). The marker according to the invention is selectively collected in organs and pathological changes.

This marker has a better contrast than iron-based markers, because it modifies the T₁ relaxation time, more advantageous in medical applications than iron which modifies mainly the T₂ relaxation time and shows a low signal and is biologically non-neutral. Low signal - here it is a strict term, especially in the context of references to the iron contrasts of MRI found in the literature review. Due to the presence of iron in the living organism, independently if the contrast is given or is not, we achieve a lower signal-to-noise ratio.

The microwave-assisted solvothermal method allows the production of biological markers in mild conditions. The substances constituting the medium during the crystallization of markers are liquids that can be selected from the biologically inert group. The use of microwaves in the production of biomarkers for use in living organisms has the advantage of maintaining the high purity conditions of the product due to the lack of contact of the resistance heating elements with the reaction mixture. Microwaves are also extremely energy-efficient in production in relation to the classical methods of obtaining metal oxides. The solvothermal processes are carried out in autoclaves intended for medium pressure operation. Using such reactors, it is easy to increase the scale of production by increasing their volume. In combination with the advantages of microwave heating, the method is good for adaptation to work in industrial conditions.

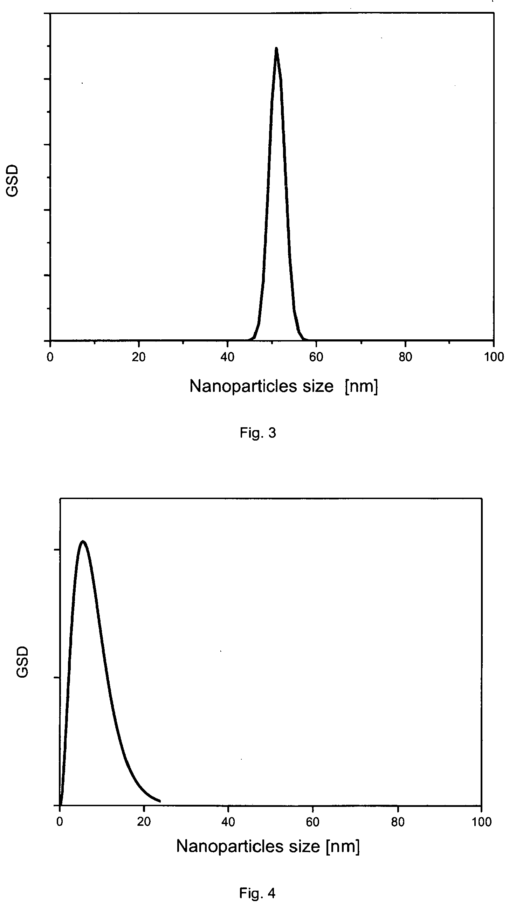

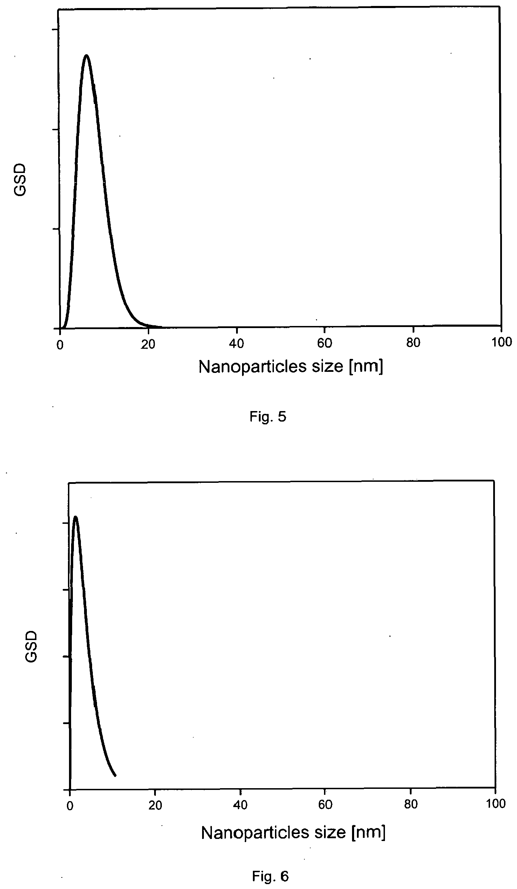

The invention will be explained in more detail in six examples. In the Figure 1 a photograph of the MRI contrast shown by the suspension of the first example is presented, Figure 2 shows the size distribution of the nanoparticles doped with Eu³⁺ ions, Figure 3 shows the particle size distribution of the Pr³⁺ doped nanoparticles, Figure 4 shows the size distribution of the undoped ZrO₂ nanoparticles, Fig. 5 shows the size distribution of nanoparticles doped with Tb³⁺ ions, Fig. 6 shows the size distribution of nanoparticles doped with Ce³⁺ ions.

### Example 1

First, ZrO₂ nanoparticles doped with 2.9 mol% europium were produced. These nanoparticles were produced by microwave-assisted solvothermal method. For this purpose, 12 g of zirconyl nitrate (V) and 0.5 g of europium (III) nitrate were dissolved in 500 mL of distilled water. Then 500 mL of absolute ethanol was added to the solution and the pH was increased by instilling 25% aqueous ammonia solution. A mixture of pH = 10 was obtained, then the liquid fraction was filtered off and the obtained precipitate was rinsed in distilled water and subjected to a microwave-assisted solvothermal process. This process was carried out at a pressure of 6 MPa, in 96% ethanol for 30 minutes. The resulting product was dried and ground until a powder was obtained. The dry powder was then dispersed in water and a suspension containing 1 mg of powder in 1 mL of water was formed.

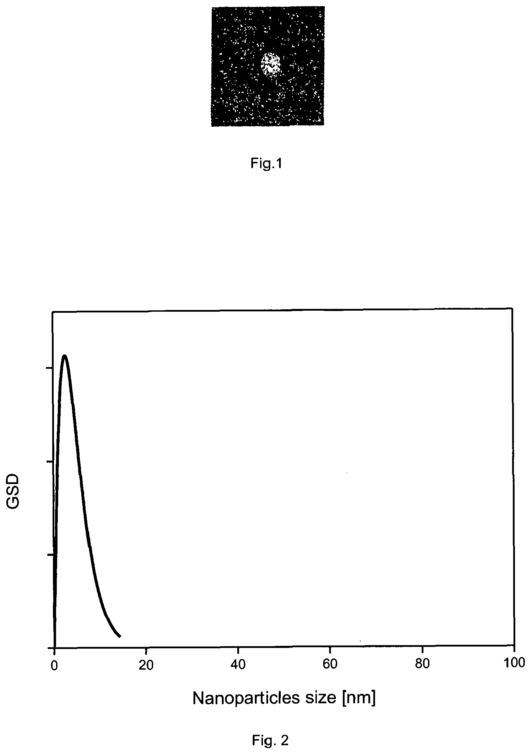

The obtained biostable marker is in the form of a suspension, in which the carrier substance are the microwave-assisted solvothermal method obtained ZrO₂ nanoparticles doped with 2.9 mol% europium, and the active substance is ethanol, the amount of active substance relative to the amount of carrier substance being 10 % w/w. The photograph (Figure 1) shows the contrast in MRI as shown by the resulting suspension.

### Example 2

First, we started to produce ZnO nanoparticles doped with 0.5 mol% praseodymium. These nanoparticles were produced by microwave-assisted solvothermal method. For this purpose, 15 g of zinc nitrate and 0.1 g of praseodymium (III) nitrate were dissolved in 200 mL of distilled water. Then, the pH of the solution was changed (in order to obtain a solution with pH = 8), in order to achieve this, a 1 molar KOH solution was suddenly added. The liquid fraction was then filtered off and the resulting precipitate was rinsed in isopropanol three times and then subjected to a microwave-assisted solvothermal process. For this purpose, 50 mL of distilled water and 2 mL of methanol were added to the obtained precipitate, then placed in the reactor container. The process was carried out at a pressure of 4 MPa for 2 hours. After the end of the process, the product was dried and ground to obtain a powder. A suspension was then made from the obtained powder and methanol, the powder content in the suspension being 0.1 µg powder/mL methanol. For purification, the mixture was subjected to 300 W ultrasound in an ultrasonic washer.

The marker obtained is in the form of a suspension in which the carrier substance is the ZnO doped with praseodymium obtained by the microwave-assisted solvothermal method, and the active substance is methanol, the amount of active substance in relation to the carrier substance being 2% w/w.

### Example 3

First, the production of pure ZrO₂ nanoparticles was started. These nanoparticles were prepared in the following way: to 1000 mL of distilled water 16 grams of zirconium chloride was added, resulting in a clear solution. The solution was alkalized with an aqueous 10 molar NaOH solution until pH = 11. The resulting precipitate was rinsed in distilled water, then twice in hexanol. The liquid fraction was filtered and the resulting precipitate was placed in a microwave-assisted solvothermal reactor and 20 mL of ethanol was added. The mixture was then subjected to a pressure of 10 MPa for 60 minutes. After the end of the process, the precipitate was drained from the mother solution and dried for 12 hours at 60°C. The obtained product was ground in a mortar until a homogeneous powder was obtained, after this the powder was dispersed in benzene using an ultrasonic homogenizer to obtain a stable suspension.

The obtained marker is in the form of suspension in which the carrier substance is zirconium oxide nanoparticles (ZrO₂) obtained by the solvothermal method, while the active substance is benzene, the amount of active substance in relation to the carrier substance is 0.5 % w/w.

### Example 4

First, the production of pure ZrO₂ nanoparticles was started. These nanoparticles were prepared in the following way: to 1000 mL of distilled water 16 grams of zirconium chloride was added to obtain a clear solution. The solution was alkalized with an aqueous 10 molar NaOH solution until pH = 11. The resulting precipitate was rinsed in distilled water, then twice in hexanol. The liquid fraction was filtered and the resulting precipitate was placed in a microwave-assisted solvothermal reactor and 20 mL of ethanol was added. The mixture was then subjected to a pressure of 6 MPa for 60 minutes. After the end of the process, the precipitate was drained from the mother solution and dried for 12 hours at 60°C. The obtained product was ground in a mortar until a homogeneous powder was obtained, after this the powder was pressed in a press under pressure of 500 kPa to obtain tablets.

The obtained marker is in the form of tablet in which the carrier substance is zirconium oxide nanoparticles (ZrO₂) obtained by the microwave-assisted solvothermal method, while the active substance is ethanol, the amount of active substance in relation to the carrier substance is 7% w/w.

### Example 5

First, the ·ZrO₂ nanoparticles doped with 0.02 mol% terbium were produced. These nanoparticles were produced by microwave-assisted solvothermal method. For this purpose, 24 g of zirconyl nitrate (V) and 0.02 g of terbium (III) nitrate (V) were dissolved in 1000 mL of distilled water. Then 200 mL 96% ethanol was added to the solution and the pH was increased by instilling 25% aqueous ammonia solution. A mixture of pH = 11 was obtained, then the liquid fraction was filtered off and the resulting precipitate was rinsed in ethanol and subjected to a microwave-assisted solvothermal process. This process was carried out at a pressure of 8 MPa, in 96% ethanol for 90 minutes. The resulting product was dried and ground until a powder was obtained. The dry powder was then dispersed in water and a suspension containing 1 mg of powder in 10 mL of water was formed.

The obtained biostable marker is in the form of a suspension, in which the carrier substance are obtained by the microwave-assisted solvothermal method ZrO₂ nanoparticles doped with 0.02 mol% terbium and the active substance is ethanol, the amount of active substance in relation to the amount of carrier substance being 5% w/w.

### Example 6

First, ZrO₂ nanoparticles were doped with 20 mol% of cerium. These nanoparticles were produced by microwave-assisted solvothermal method. For this purpose, 24 g of zirconyl nitrate (V) and 7 g of cerium (III) nitrate were dissolved in 100 mL of distilled water. Then 100 mL of absolute ethanol was added to the solution and the pH was increased by instilling 25% aqueous ammonia solution. A mixture of pH = 10 was obtained, then the liquid fraction was filtered off and the obtained precipitate was rinsed in distilled water and subjected to a microwave-assisted solvothermal process. This process was carried out at a pressure of 4 MPa in water for 50 minutes. The resulting product was dried and ground until a powder was obtained. The dry powder was then dispersed in water and a suspension containing 1 mg of powder in 1 mL of water was formed.

The biostable marker obtained is in the form of a suspension in which the carrier substance are ZrO₂ nanoparticles doped with 20 mol% cerium obtained by the microwave-assisted solvothermal method, and the active substance is ethanol, the amount of active substance relative to the amount of carrier substance being 3% w/w.

## Claims

1. Biostable, active in nuclear magnetic resonance marker in the form of a powder, tablet or suspension, containing a carrier substance and an active substance, **characterized in that** the carrier substance is zirconium oxide nanoparticles (ZrO₂) and/or zinc oxide nanoparticles (ZnO) obtained by the microwave solvothermal method whereas the active substance is a solvent, wherein it contains the active substance in an amount of from 0.5% m/m of the marker to 10% m/m of the marker.

2. Biostable marker according to claim 1, **characterized in that** the carrier substance, obtained by the microwave solvothermal method, is zirconium oxide nanoparticles (ZrO₂) and/or zinc oxide nanoparticles (ZnO) and lanthanide ions, preferably such as: La³⁺, Ce³⁺, Pr³⁺, Nd³⁺, Sm³⁺, Eu³⁺, Tb³⁺, Ho³⁺, Er³⁺, Tm³⁺, Yb³⁺, Lu³⁺ in an amount of 0.01% to 20 mol%.

3. Biostable marker according to claims from 1 to 2, **characterized in that** the lanthanide ion is selected from the group consisting of: Eu³⁺, Pr³⁺, Tb³⁺, Ce³⁺.

4. Biostable marker according to claims 1 to 3, **characterized in that** it is obtained by a microwave-assisted solvothermal method from a transition metal solution having a concentration of a transition metal in a solvent solution of 0.001 mol/m³ to 5 mol/dm³, wherein the transition metal solution also contains ammonia water and solvent and the pH of the solution is from 6 to 11, and the precipitate obtained from the solution is rinsed with solvent, and the microwave assisted solvothermal process is carried out in the pressure range from 2 MPa to 10 MPa in a time not shorter than 1s.

5. Biostable marker according to claims from 1 to 4, **characterized in that** the particle size of the powder is in the range from 1 nm to 100 nm.

6. Biostable marker according to the process of any one of claims 1 to 5, wherein the solvent is selected from the group consisting of: water, alcohol or physiological salt.

7. Biostable marker according to claims 1, 2, 3, 4, 5, or 6, **characterized in that** the minimum powder concentration in the suspension is 0.001 µ/l.

8. Use of a biostable marker as defined in any of claims 1 to 7 as contrast in magnetic resonance imaging.
